# EUROPEAN PATENT APPLICATION

(11) **EP 2 031 372 A1**
(43) Date of publication of application: **04.03.2009**
(21) Application number: 08015031.1
(22) Date of filing: 26.08.2008
(51) Int. Cl.: G01N 21/21, G01N 33/543, G01N 33/552, G01N 33/557

(54) **Device for determining the concentration of a substance present in a solution, using ellipsometry**

(30) Priority: 30.08.2007 NL 1034311
(71) Applicant: Delbia B.V., 1951 NH Velsen-Noord (NL)
(72) Inventor: Hermens, Willem Theodoor, 6221 JN Maastricht (NL)
(74) Representative: Valkonet, Rutger

(57) **Abstract**

The invention relates to a device for determining the concentration of a substance present in a solution, using ellipsometry.

Said device is used for determining the rate of precipitation, which is a measure of the concentration of the substance to be determined. The object of the invention is to provide an improved measuring technique based on optical ellipsometry or another technique for measuring the mass bonded to a solid surface (substrate), such as "surface plasmon resonance" (SPR), "quartz crystal microbalance" (QCM) etc, by means of which the concentration of a substance present in a liquid, which liquid is in contact with the substrate, can be measured with a higher degree of accuracy.

According to the invention, the device is to that end **characterised in that** said substance is glucose, wherein the substrate comprises glucose oxidase (GOX) and/or horseradish peroxidase (HRP) bonded thereto, and wherein said means are arranged for determining the presence of glucose on the basis of the precipitation rate of diaminobenzene (DAB) on the substrate as measured during operation.

## Description

The invention relates to a device for determining the concentration of a substance present in a solution, using ellipsometry, comprising
a measuring space for receiving the solution;
a substrate disposed in said measuring space;
a light-emitting element for emitting light to the substrate disposed in the measuring space;
a first polarizer disposed between the light-emitting element and the substrate;
a compensator disposed between the first polarizer and the substrate;
a light-absorbing element for detecting light reflected by the substrate;
a second polarizer disposed between the substrate and the light-absorbing element; as well as
means for determining the concentration of the substance present in the liquid on the basis of the polarisation change between the emitted light and the reflected light effected by the substances bonded to the substrate.

Ellipsometry, and in particular optical ellipsometry, is a nondestructive optical method by which the dielectric properties of the material are measured on the basis of the change of the polarisation state of a bundle of laser light before and after the reflection from said material surface. More specifically, the refractive index and the thickness of a layer adsorbed on the material surface in question can be determined by means of the optical ellipsometry measuring technique. Think in this regard of a measurement setup in which a surface is coated with a thin layer of matter, for example an oxide layer, a lipid membrane or a layer of adsorbed proteins, whereupon a beam of laser light is directed at the surface in question, after which the polarisation change of the laser light before and after the reflection is determined.

In the measuring technique in which a layer of adsorbed proteins is used (so-called biological applications), it may be important that the reflecting surface may be present not only in the air but also in water or in buffer solutions to which the lipids and proteins can be added.

The object of the invention is to provide an improved measuring technique based on optical ellipsometry or another technique for measuring the mass bonded to a solid surface (substrate), such as "surface plasmon resonance" (SPR), "quartz crystal microbalance" (QCM) etc, by means of which the concentration of a substance present in a liquid, which liquid is in contact with the substrate, can be measured with a higher degree of accuracy.

According to the invention, the device is to that end characterised in that said substance is glucose, wherein the substrate comprises glucose oxidase (GOX) and/or horseradish peroxidase (HRP) bonded thereto, and wherein said means are arranged for determining the presence of glucose on the basis of the precipitation rate of diaminobenzene (DAB) on the substrate as measured during operation.

In another embodiment, the solution comprises glucose oxidase (GOX), and more in particular the horseradish peroxidase (HRP) is bound to the substrate surface, whilst in another embodiment the solution comprises horseradish peroxidase (HRP), and more in particular the glucose oxidase (GOX) is bound to the substrate surface.

In a special embodiment, the substrate is made of silicon. It is possible to obtain a substrate having a silicon surface exhibiting a high degree of purity, which has a positive effect on the accuracy of the measurement. In addition to that, the silicon substrate can be easily cleaned by means of an etching technique for carrying out a measurement.

According to the invention, the silicon substrate may have been pre-treated with chromium acid in that case, and be provided with a layer of polyvinyl chloride (PVC). To realise an effective measurement, the thickness of the PVC layer may be about 5 - 25 nm, in particular about 15 nm.

In another functional embodiment, the silicon substrate is provided with a multiple layer built up of alternating layers of concanavalin and glucose oxidase (GOX). Preferably, the outermost layer may consist of horseradish peroxidase (HRP) in that case.

In another functional embodiment, the thickness of the precipitate ranges between 5 and 50 nm, and the refractive index ranges between 1.35 and 1.65.

To obtain an accurate measurement, the device may be provided with stirring means for stirring the glucose-containing liquid that is present in the measuring space.

The invention will now be explained in more detail with reference to the drawings, in which:
Figure 1 shows a schematic diagram of an ellepsometer setup;
Figure 2 schematically shows the effect of the glucose reaction in a device according to the invention.

For a better understanding of the invention, like parts will be indicated by identical numerals in the various figures and the description thereof below.

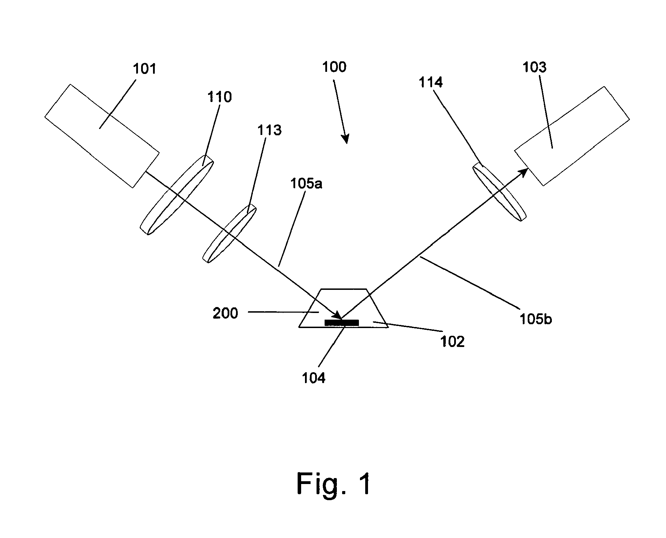

Figure 1 shows an embodiment of an ellipsometer 100 according to the prior art. The ellipsometer is made up of a light source, in this case a laser 101, a measuring space or cuvette 102, as well as a light-absorbing element 103. The laser 101 emits a light beam 105 in the direction of the measuring space 102, in which measuring space 102 the substrate 104 to be examined or the solution 200 is present. The incident light passes a polarizer 110 and a compensator 113, whilst the reflected light passes a second polarizer 114. On the basis of the reflection of the incident light 105a, as well as the differences or the change in the polarisation state of the incident light 105a and the reflected light 105b, it is possible to determine specific material properties of the substances present in the measuring space 102, which substances are bonded to the substrate 104.

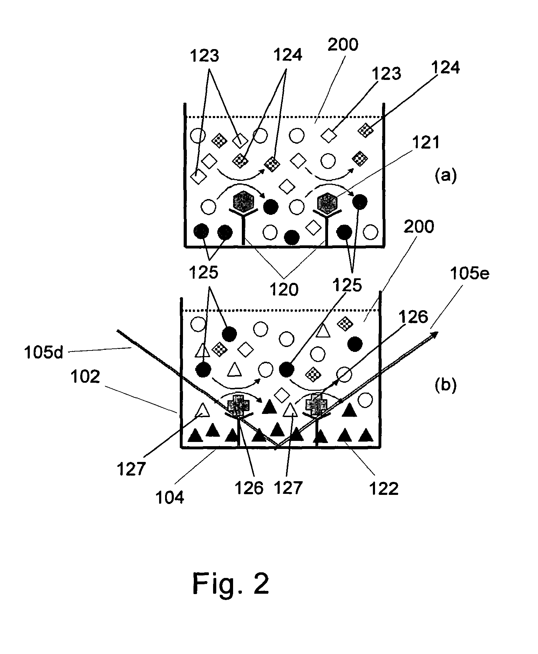

The invention is shown in figure 2, in which numeral 102 indicates a measuring space which is filled with an aqueous solution or a buffer solution 200. Various substances have been added to the solution 200, among which GOX and HRP, and one of said enzymes, or both, is (are) bonded to the substrate surface 104, possibly via so-called bonding agents 120, for example concanavalin A or antibodies.

The measuring technique according to the invention makes use of the reaction that takes place in the measuring space 102, with the solution in the measuring space containing glucose. The substrate 104 is provided with a precipitate 122 formed by means of GOX and HRP during the measurement, which precipitates on the substrate 104 at a rate proportional to the amount of glucose that is present.

This embodiment of the measuring device 100 has the additional advantage that the precipitation on the substrate 104 is measured while the precipitation reaction of the precipitate being formed on the substrate 104 is still taking place in the solution 200. Thus, measuring results can be obtained without time-consuming rinsing steps being required.

In subfigure (a) of figure 2, a first step of the measuring method is being carried out, in which the glucose 123 present in the solution 200 is converted into gluconolactone 124 by the GOX 121 bonded to the substrate 104 via the bonding agents 120, with hydrogen peroxide H₂O₂ 125 being formed as a by-product. Subfigure (b) of figure 2 shows a second step. In this step, the soluble DAB 126 present in the solution 200 is converted into precipitating DAB 122 by the HRP 127 bonded to the substrate 104 via the bonding agents 120 and the hydrogen peroxide H₂O₂ 125 formed as a by-product in step (a).

The two reactions shown in the subfigures (a) and (b) can take place simultaneously on a substrate surface 104 to which both glucose oxidase (GOX) and horseradish peroxidase (HRP) are bonded.

It is furthermore noted that it is also possible to use a different technique, for example SPR or QCM, instead of an ellipsometry technique for measuring precipitation on the substrate. Nor is it necessary to obtain the precipitate by means of HRP and DAB, it is also possible to use a different combination of enzyme and converted substance.

## Claims

1. A device for determining the concentration of a substance present in a solution, using ellipsometry, comprising
a measuring space for receiving the solution;
a substrate disposed in said measuring space;
a light-emitting element for emitting light to the substrate disposed in the measuring space;
a first polarizer disposed between the light-emitting element and the substrate;
a compensator disposed between the first polarizer and the substrate;
a light-absorbing element for detecting light reflected by the substrate;
a second polarizer disposed between the substrate and the light-absorbing element; as well as
means for determining the concentration of the substance present in the liquid on the basis of the polarisation change between the emitted light and the reflected light effected by the substances bonded to the substrate, **characterised in that** said substance is glucose, wherein the substrate comprises glucose oxidase (GOX) and/or horseradish peroxidase (HRP) bonded thereto, and wherein said means are arranged for determining the presence of glucose on the basis of the precipitation rate of diaminobenzene (DAB) on the substrate as measured during operation.

2. A device according to claim 1, **characterised in that** the solution contains GOX and/or HRP.

3. A device according to claim 1 or 2, **characterised in that** said GOX and/or HRP are bound to the substrate surface.

4. A device according to any one or more of the preceding claims, **characterised in that** said substrate is made of silicon.

5. A device according to claim 4, **characterised in that** said silicon substrate has been pre-treated with chromium acid.

6. A device according to claim 5, **characterised in that** said silicon substrate is provided with a layer of polyvinyl chloride (PVC).

7. A device according to claim 6, **characterised in that** the thickness of the PVC layer is about 5 - 25 nm, in particular about 15 nm.

8. A device according to any one or more of the preceding claims, **characterised in that** the silicon substrate is provided with a multiple layer built up of alternating layers of concanavalin and glucose oxidase (GOX).

9. A device according to claim 8, **characterised in that** the outermost layer consists of horseradish peroxidase (HRP).

10. A device according to any one or more of the preceding claims, **characterised in that** the thickness of the precipitate ranges between 5 and 50 nm, and the refractive index ranges between 1.35 and 1.65.

11. A device according to any one or more of the preceding claims, **characterised in that** the device is provided with stirring means for stirring the glucose-containing liquid that is present in the measuring space.
